Europäisches Patentamt

European Patent Office

Office européen des brevets

(19)

(11) Veröffentlichungsnummer: **0 283 928 B1**

(12) **EUROPÄISCHE PATENTSCHRIFT**

(45) Veröffentlichungstag der Patentschrift: **17.06.92**

(51) Int. Cl.⁵: **A61B 19/02**, A61L 2/26

(21) Anmeldenummer: **88104234.5**

(22) Anmeldetag: **17.03.88**

(54) **Aufnahmeelement, insbesondere für die Medizintechnik.**

(30) Priorität: **26.03.87 DE 3710016**

(43) Veröffentlichungstag der Anmeldung:
**28.09.88 Patentblatt 88/39**

(45) Bekanntmachung des Hinweises auf die
Patenterteilung:
**17.06.92 Patentblatt 92/25**

(84) Benannte Vertragsstaaten:
**AT BE CH DE ES FR GB GR IT LI LU NL SE**

(56) Entgegenhaltungen:
**DE-A- 2 713 094**
**US-A- 4 043 754**
**US-A- 4 135 868**

(73) Patentinhaber: **Wolf, Hans-Joachim**
**Theodor-Schweitzer-Strasse 1**
**W-7137 Sternenfels 1(DE)**

(72) Erfinder: **Wolf, Hans-Joachim**
**Theodor-Schweitzer-Strasse 1**
**W-7137 Sternenfels 1(DE)**

(74) Vertreter: **Frank, Gerhard, Dipl.-Phys.**
**Patentanwälte Dr. F. Mayer & G. Frank Westliche 24**
**W-7530 Pforzheim(DE)**

## Beschreibung

Die Erfindung betrifft ein Aufnahmeelement, insbesondere für die Medizintechnik, das zur Lagerung und Fixierung eines zu sterilisierenden Gegenstandes, beispielsweise eines Operationsbestecks, in einen Trägereinsatz einsteckbar ist, der hierzu eine Vielzahl von Einstecköffnungen für mehrere derartige Aufnahmeelemente für unterschiedliche Gegenstände aufweist.

Derartige Aufnahmeelemente sind aus der US-PS 4,135,868 bekannt. Dort ist in einem kofferähnlichen Aufbewahrungsgegenstand ein Trägereinsatz eingelassen, in dessen Öffnungen reiterähnliche Einsteckelemente einsteck- und einrastbar sind, die ihrerseits kammartig ausgebildete Lagerelemente oder Brückenteile aufnehmen, auf die bzw. in die beispielsweise Operationsbestecke eingelegt bzw. aufgelegt werden können.

Zur lagegerechten Fixierung dieser Bestecke sieht die vorbekannte Vorrichtung vor, daß ähnliche Aufnahmeelemente auch in einen im Deckel dieses kofferähnlichen Aufbewahrungsgegenstandes eingelassen und Trägereinsatz eingesteckt sind, die aber anstelle der brücken-oder reiterähnlichen Unterlagen elastische Teile, beispielsweise Schaumstoffblöcke, tragen, derart, daß beim Zuklappen des Deckels diese elastischen Halterungteile die zu haltenden Besteckteile auf deren Unterlage fixieren.

Es versteht sich von selbst, daß diese Fixierung nur bei geschlossenem Deckel des Instrumentenkoffers erreicht werden kann. Dabei bewirkt das Öffnen des Deckels zwangsläufig die Freigabe sämtlicher dort gehaltenen Instrumente, es handelt sich sozusagen also um eine "kollektive" Sicherung der zu sterilisierenden Gegenstände in ihren Positionen auf dem Trägereinsatz. Die gezeigten Aufnahmeelemente sind auch nur bedingt für medizinische Instrumente geeignet, wie zum Beispiel Endoskope, die räumlich kompliziert aufgebaut sind, beispielsweise aus verschiedenen Teilen unterschiedlichen Durchmessers und verschiedener Formgebung bestehen.

Relativ kurze, ringförmige Teile mit größerem Durchmesser lassen sich beispielsweise mit den vorbekannten Aufnahmeelementen nicht zufriedenstellend fixieren.

Aufgabe der Erfindung ist es daher, diese Aufnahmeelemente so weiterzubilden, daß sie eine größere Anpassungsfahigkeit an unterschiedliche Geräteformen besitzen und eine individuelle Halterung der einzelnen Teile ermöglicht wird.

Erfindungsgemäß wird diese Aufgabe dadurch gelöst, daß ein Aufnahmeelement aus mindestens einem zumindest teilweise profilierten Halteblock und mindestens einem Haltebügel und/oder Lagerbügel gebildet ist, und daß Haltebügel/Lagerbügel mindestens eine Öffnung besitzen, mittels der sie über mindestens einen der Halteblöcke steckbar und die Kanten ihrer Öffnungen unter elastischer Verformung des Halte/Lagerbügels in rastenden Eingriff mit der Profilierung des Halteblocks/der Halteblöcke bringbar sind.

Im Unterschied zur vorbekannten Ausführungsform der Aufnahmeelemente nach der US-PS 4,135,868 ermöglichen diese Aufnahmeelemente eine individuelle Halterung und Fixierung der ihnen zugeordneten Geräteteile, wobei insbesondere eine einfache Anpassung an die Dicke bzw. Höhe des jeweiligen Teils dadurch erzielbar ist, daß der Haltebügel mehr oder weniger tief über den Halteblock gedrückt wird.

In vorteilhafter Ausgestaltung der Erfindung ist vorgesehen, daß der Halteblock ein zylindrisches Formteil ist, an dessen Umfang eine Mehrzahl übereinanderliegender Rastrippen ausgebildet ist, und daß der Haltebügel oder Lagerbügel aus elastischem kunststoffmaterial mit rechteckigem Querschnitt gebildet ist.

Bei dieser Ausgestaltung ist eine einfache Fertigung der beiden das erfindungsgemäße Aufnahmeelement bildenden Teile gewährleistet, wobei durch einfache Variationen von Haltebügel oder Lagerbügel und Kombinationen solcher Teile auf einem oder zwei Halteblöcken eine Vielzahl von Aufnahmeelementen mit einem Minimum an Grundbauteilen realisierbar ist. Das erfindungsgemäße Zusammenwirken von Halteblock und Haltebügel oder Lagerbügel, d.h. deren elastische Verrastung in beliebiger Höhenposition, ermöglicht daher die Halterung beliebig geformter oder zusammengesetzter medizinischer Instrumente an den hierfür notwendigen Stellen im Trägereinsatz, wobei dieser hinsichtlich seiner Fläche optimal genutzt werden kann, da die Aufnahmeelemente nur ein Minimum an Platz beanspruchen.

Besonders hervorzuheben ist die extrem leichte Handhabbarkeit dieser Aufnahmeelemente, sowohl das Verriegeln des Aufnahmeelementes zur Fixierung eines Geräteteils, als auch das Entriegeln kann mit einem einzigen Handgriff bewerkstelligt werden, im wesentlichen durch einen Fingerdruck auf die hierfür vorgesehenen Stellen der Haltebügel oder Lagerbügel.

Weitere Ausgestaltungen der Erfindung sind weiteren abhängigen Ansprüchen zu entnehmen.

Mehrere Ausführungsbeispiele erfindungsgemäßer Aufnahmeelemente werden nun anhand von Zeichnungen näher erläutert, es zeigen:

Figur 1: Einen Querschnitt durch ein in den Trägereinsatz eingesetztes Aufnahmeelement in der Ebene I-I der Figur 2,

Figur 2: eine Aufsicht auf das eingesetzte Trägerelement der Figur 1,

Figur 3: eine Seitenansicht eines Aufnahmeelementes mit zwei Haltebügeln,

Figur 4: einen Querschnitt durch ein Aufnahmeelement mit symmetrischem Haltebügel in der Ebene IV-IV der Figur 5,

Figur 5: eine Aufsicht auf das Aufnahmeelement der Figur 4, und

Figur 6: eine Schnittdarstellung durch ein Aufnahmeelement, gebildet aus zwei Halteblöcken und je einem beide Halteblöcke übergreifenden Haltebügel und Lagerbügel.

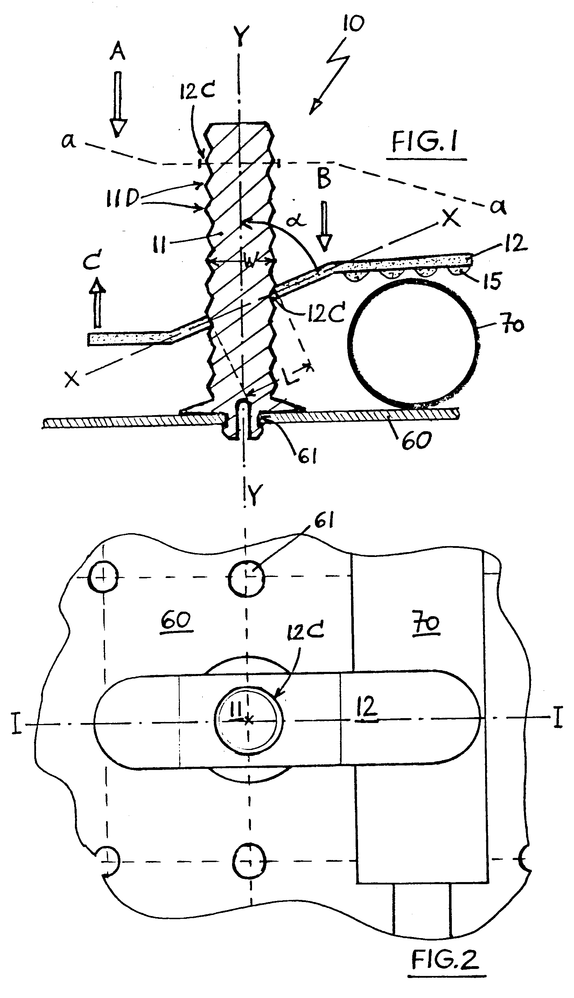

Das Prinzip der erfindungsgemäßen Aufnahmeelemente wird anhand der Figuren 1 und 2 detailliert erläutert:

Das erfindungsgemäße Aufnahmeelement 10 gemäß dem Ausführungsbeispiel der Figuren 1 und 2 besteht aus lediglich zwei Teilen, nämlich einem Halteblock 11 und einem Haltebügel 12.

Der Halteblock 11 ist als zylindrisches Formteil ausgebildet, das in rasterartig eingebrachten Öffnungen 61 einer Trägerplatte 60 einrastbar ist. Hierzu ist der Fußteil des Halteblocks 11 in seiner Längsachse Y-Y geschlitzt, so daß die beiden gegenüberliegenden Teile beim Einstecken in die Öffnung 61 zusammenfedern und nach Durchtritt durch die Öffnung wieder auseinanderfedern, wobei Rastschultern die Trägerplatte 60 dann hintergreifen. Im wesentlichen entspricht diese Art der Verankerung der Verankerung der Aufnahmeelemente gemäß der US-PS 4,135,868, so daß hierauf nicht besonders eingegangen wird.

Zur Stabilisierung des Halteblocks 11 in seiner vertikalen Position auf der Trägerplatte 60 ist dessen Fußteil konusförmig verbreitert.

An seinem Umfang weist der zylindrische Halteblock 11 horizontal umlaufende Rippen 11D auf, deren Funktion die eines Gegenlagers zum Haltebügel 12 ist, wie weiter unten noch im einzelnen erläutert wird.

Der Haltebügel 12 besteht aus einem elastisch verformbaren Kunststoffteil mit einem Mittelbereich, in dem eine Öffnung 12C eingebracht ist und zwei gegenüber dem Mittelbereich leicht abgewinkelten Endbereichen, von denen der eine Endbereich zur Halterung eines medizinischen Objektes 70 dient und der andere Endbereich zur Betätigung des Haltebügels.

Von besonderer Bedeutung ist die Dimensionierung dieser Öffnung 12C im Verhältnis zum Querschnitt des Halteblocks 11:

Die lichte Weite L der Öffnung 12C in der Ebene I-I (Längsebene des Haltebügels 12) muß geringfügig größer bemessen sein als der größte Durchmesser W des Halteblocks 11, um ein einfaches Überlegen des Haltebügels 12 über den Halteblock 11 einerseits und eine sichere Verrastung andererseits zu ermöglichen. Damit wird erreicht, daß der Halteblock 11 den Haltebügel 12 freigibt, wenn die Ebene der Öffnung 12C praktisch senkrecht auf der Längsachse Y-Y des Halteblocks 11 steht, da dann beidseitig der Rippen 11D noch ausreichend Spiel für eine Vertikalbewegung des Haltebügels 12 gegeben ist. Daraus ergibt sich andererseits, daß bei einer Verkantung dieses Mittelbereichs des Haltebügels 12 gegenüber der Längsachse Y-Y des Halteblocks 11, d.h. wenn der in Figur 1 dargestellte Winkel α kleiner als 90° ist, eine Position erreicht wird, bei der die Projektion der lichten Weite L in die Horizontale den größten Durchmesser W des Halteblocks 11 unterschreitet, so daß dann ein Klemm- oder Rasteffekt auftritt, der unter der Wirkung des elastischen Kunststoffmaterials bewirkt, daß der Haltebügel 12 auf dem Halteblock 11 blockiert ist, wobei eine der Profilrippen 11D als Gegenlager für eine Umfangskante der Öffnung 12C dient.

Aus diesen Prinzipien ergibt sich die Handhabung des dargestellten Aufnahmeelementes wie folgt:

Zur Arretierung eines medizinischen Objektes 70 wird zunächst der Haltebügel 12 in einer Position über den Halteblock 11 gelegt, wie diese durch die gestrichelte Linie a-a dargestellt ist. Da hierbei der Mittelbereich des Haltebügels 12 senkrecht zur Längsachse Y-Y liegt, rutscht der Haltebügel folglich nach unten, bis die mit Haltenoppen 15 versehene Unterseite des Halteteils auf dem Objekt 70 aufliegt (diese Position ist nicht dargestellt). Wenn dann die Bedienungsperson in Richtung des Pfeiles A auf das gegenüberliegende Ende des Haltebügels 12 drückt, so bewegt sich der Haltebügel unter Verkantung des Öffnungsbereiches weiter nach unten, wobei gleichzeitig der Druck auf das zu sichernde Objekt 70 verstärkt wird. Schließlich wird die in Figur 1 ausgezogen dargestellte Endposition des Haltebügels 12 erreicht, wo die Ebene X-X der Öffnung 12C einen bestimmten Winkel α mit der Längsachse Y-Y des Halteblocks 11 bildet und die Verrastung eintritt. Durch entsprechenden Kraftaufwand in Richtung des Pfeiles A lassen sich in der Regel zwei bis drei Rastpositionen auf den Rastrippen 11D erreichen, die infolge der Elastizität des Kunststoffmaterials des Haltebügels 12 einen mehr oder weniger starken Haltedruck auf das zu sichernde Objekt 70 bewirken.

Zur Lösung dieser Verriegelung ist es allgemein gesprochen erforderlich, die Ebene X-X wieder senkrecht zur Längsache Y-Y zu bringen, was beispielsweise durch Druck in Richtung des Pfeiles B, gegebenenfalls durch gleichzeitigen Zug in Richtung des Pfeiles C, erreicht werden kann, so daß dann der Haltebügel 12 wieder in die gestrichelt dargestellte Position a-a bewegt und gegebenenfalls vom Halteblock 11 abgenommen werden kann.

Alternativ zu dieser Möglichkeit ist auch vorstellbar, den Halteblock 11 mit einem elastischen, d.h. schwenkbaren Fußteil zu versehen, so daß durch eine Neigung des Halteblocks 11 in Figur 1 nach links ebenfalls die Ebene X-X der Öffnung 12C senkrecht zur Längsachse Y-Y gebracht werden könnte, was wie oben geschildert, Voraussetzung für die Lösung der Arretierung ist.

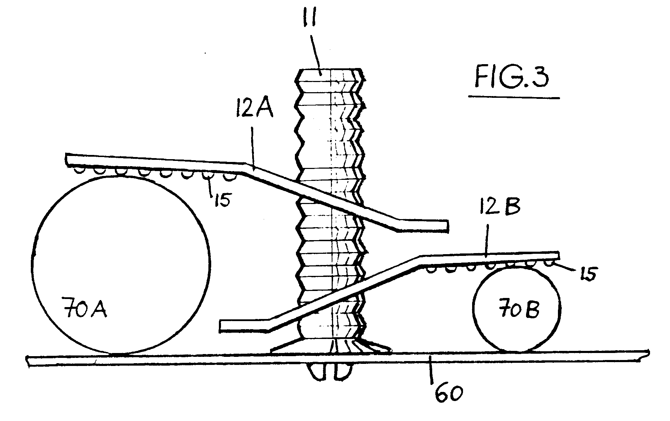

Die Figuren 3 bis 6 zeigen weitere Ausführungsbeispiele, die unter Heranziehung des geschilderten Prinzips es ermöglichen, mit unterschiedlich gestalteten Haltebügeln und Lagerteilen eine beliebige, individuelle Halterung verschiedenster Teile zu bewerkstelligen.

So zeigt Figur 3 die Verwendung von zwei Haltebügeln 12A,12B auf einem gemeinsamen Halteblock 11 zur Halterung von Gegenständen 70A,70B verschiedenen Durchmessers. Es ist klar ersichtlich, daß auch die Winkelposition der beiden Haltebügel 12A,12B zueinander in weitem Rahmen variierbar ist.

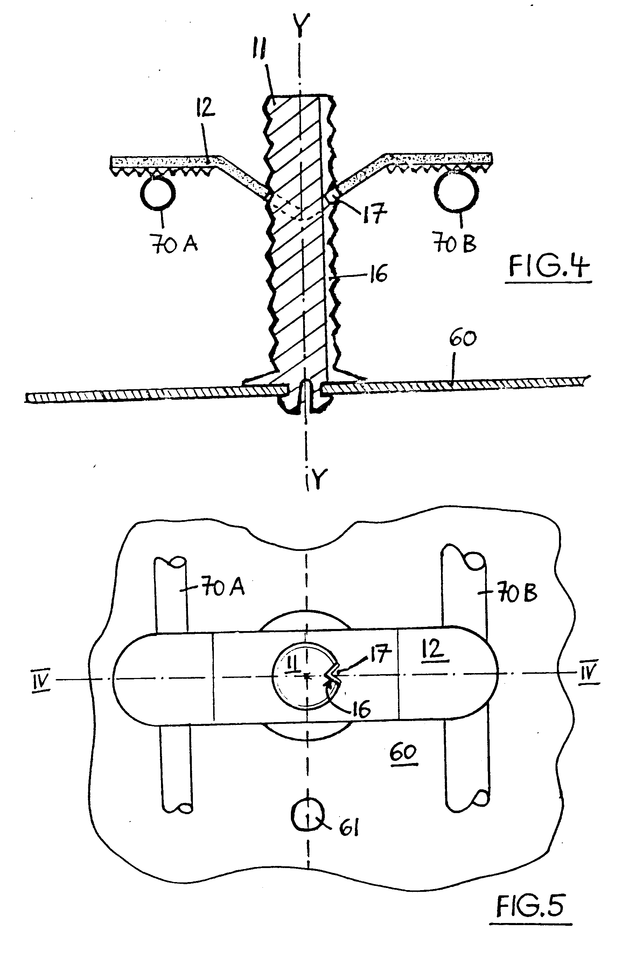

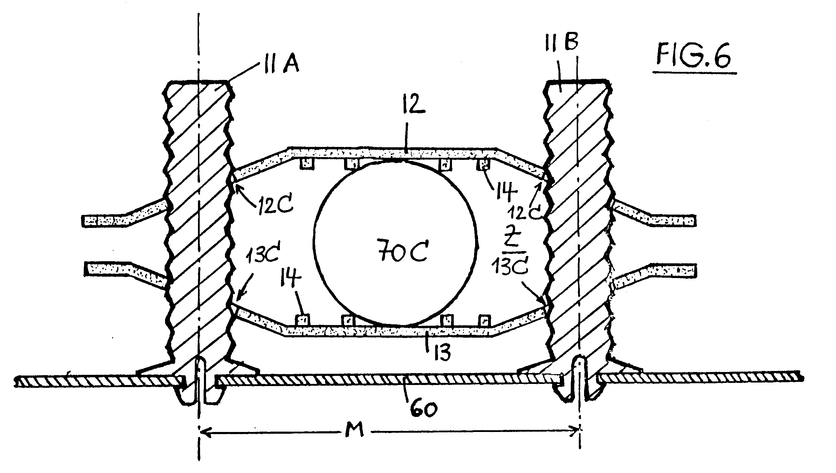

Beim Ausführungsbeispiel der Figur 4 und 5 ist der Haltebügel symmetrisch ausgeführt, die Öffnung 12C befindet sich also im V-förmig abgewinkelten Mittelteil des Haltebügels 12. Zusätzlich ist bei diesem Ausführungsbeispiel vorgesehen, daß der Halteblock 11 eine vertikale Längsnut 16 aufweist, in die eine Führungsnase 17 des Haltebügels 12 eingreift. Damit kann eine bestimmte Richtungsorientierung des Haltebügels 12 relativ zum Trägereinsatz 60 vorgegeben werden, wenn dies Art und Raumform der zu haltenden Objekte 70A,70B als zweckmäßig erscheinen läßt.

Beim Ausführungsbeispiel der Figur 6 ist schließlich dargestellt, wie zwei Halteblöcke 11A,11B mit je einem brückenförmigen Lagerbügel 13 und Haltebügel 12 verbunden werden können, so daß im gebildeten Zwischenraum Z zwischen Lagerbügel 13 und Haltebügel 12 ein Objekt 70C sicher gehalten ist. Da der Abstand M der Öffnungen 61 rasterartig auf dem Trägereinsatz 60 verteilt ist, läßt sich eine solche Variante ebenfalls an beliebigen Stellen des Trägereinsatzes realisieren.

Aus den geschilderten Ausführungsbeispielen wird erkennbar, daß durch die modulartige Anordnung mehrerer Halteblöcke 11 und geeigneter Haltebügel oder Lagerbügel auf dem Trägereinsatz 60 alle denkbaren Gegenstände sicher fixierbar sind, unabhängig von der jeweiligen Raumpositionierung und den Abmessungen des gehaltenen Geräteabschnittes.

**Patentansprüche**

1. Aufnahmeelement, insbesondere für die Medizintechnik, das zur Lagerung und Fixierung eines zu sterilisierenden Gegenstandes, beispielsweise eines Operationsbestecks, in einen Trägereinsatz einsteckbar ist, der hierzu eine Vielzahl von Einstecköffnungen für mehrere, derartige, Aufnahmeelemente für unterschiedliche Gegenstände aufweist, dadurch gekennzeichnet, daß ein Aufnahmeelement aus mindestens einem, zumindest teilweise profilierten Halteblock (11,11A,11B) und mindestens einem Haltebügel (12,12A,12B) und/oder Lagerbügel (13) gebildet ist, und daß die Haltebügel/Lagerbügel mindestens eine Öffnung (12C,13C) besitzen, mittels der sie über mindestens einen der Halteblöcke (11,11A,11B) steckbar und die Kanten der Öffnung (12C,13C) unter elastischer Verformung des Haltebügels/Lagerbügels in rastenden Eingriff mit der Profilierung des Halteblocks/der Halteblöcke (11,11A,11B) bringbar sind.

2. Aufnahmeelement nach Anspruch 1, dadurch gekennzeichnet, daß der Halteblock (11,11A,11B) ein zylindrisches Formteil ist, an dessen Umfang eine Mehrzahl übereinanderliegender Rastrippen (11D) ausgebildet ist.

3. Aufnahmeelement nach Anspruch 1, dadurch gekennzeichnet, daß die lichte Länge (L) der Öffnung (12C,13C) des Haltebügels/Lagerbügels geringfügig größer ist als die größte Weite (W) des Halteblocks (11,11A,11B) in der horizontalen Ebene, so daß der rastende Eingriff erreicht wird, wenn die Ebene (X-X) der Öffnung (12C,13C) gegen die Längsachse (Y-Y) des Halteblocks (11,11A,11B) um einen Winkel ($\alpha$) ungleich 90° geneigt ist.

4. Aufnahmeelement nach Anspruch 1, dadurch gekennzeichnet, daß der Haltebügel (12) /der Lagerbügel (13) aus zwei symmetrischen Bügelteilen mit einer gemeinsamen mittigen Öffnung besteht.

5. Aufnahmeelement nach Anspruch 1, dadurch gekennzeichnet, daß der Haltebügel (12) /der Lagerbügel (13) aus einem gemeinsamen Brückenteil mit zwei Öffnungen (12C,13C) in seinen Endbereichen besteht.

6. Aufnahmeelement nach Anspruch 1 bis 5, dadurch gekennzeichnet, daß Haltebügel/Lagerbügel aus elastischem Kunststoffmaterial mit rechteckigem Querschnitt gebildet ist/sind.

7. Aufnahmeelement nach Anspruch 1 bis 6, dadurch gekennzeichnet, daß die die Öffnungen (12C,13C) umgebende Fläche des Haltebügels/Lagerbügels gegenüber den Endbereichen bzw. dem Brückenteil nach oben bzw. nach unten abgewinkelt ist.

8. Aufnahmeelement nach Anspruch 5 und 7, dadurch gekennzeichnet, daß ein Haltebügel (12) und ein Lagerbügel (13) mit je gemeinsamem Brückenteil so übereinander auf zwei Halteblöcken (11A,11B) aufgesteckt sind, daß ein Zwischenraum (Z) zur Aufnahme eines zu sterilisierenden Gegenstandes (70C) gebildet wird.

9. Aufnahmeelement nach Anspruch 5 oder Anspruch 8, dadurch gekennzeichnet, daß die als Auflagefläche oder Haltefläche dienende Seite von Haltebügel oder Lagerbügel mit Zapfen (14) oder Noppen (15) versehen ist.

10. Aufnahmeelement nach Anspruch 1, dadurch gekennzeichnet, daß der Halteblock (11) und der Haltebügel (12) zur Richtungsdefinition kor-respondierende Führungsmittel, wie zum Bei-spiel eine vertikale Längsnut (16) und eine darin gleitende Führungsnase (17), aufweisen.

## Claims

1. Holder, especially for the medical field, which holder is insertable into a carrier insert member for the supporting and securing of an object to be sterilised, for example an operating instrument, which insert member includes, for such purpose, a plurality of insert apertures for a plurality of such holders for holding various objects, characterised in that a holder is formed from at least one, at least partially profiled supporting block (11, 11A, 11B) and at least one retaining bracket (12, 12A, 12B) and/or bearing bracket (13), and in that the retaining brackets/bearing brackets have at least one aperture (12C, 13C), by means of which they are mountable over at least one of the supporting blocks (11, 11A, 11B), and the edges of the aperture (12C, 13C) can be brought into locking engagement with the profiled configuration of the supporting block/supporting blocks (11, 11A, 11B) due to the resilient deformation of the retaining bracket/bearing bracket.

2. Holder according to claim 1, characterised in that the supporting block (11, 11A, 11B) is a cylindrically shaped part, a plurality of locking ribs (11D), which are situated one above the other, being provided on the periphery of said shaped part.

3. Holder according to claim 1, characterised in that the inside length (L) of the aperture (12C, 13C) in the retaining bracket/bearing bracket is slightly greater than the greatest width (W) of the supporting block (11, 11A, 11B) in the horizontal plane, so that the locking engagement is achieved when the plane (X-X) of the aperture (12C, 13C) is inclined about an angle ($\alpha$), which is not equal to 90°, relative to the longitudinal axis (Y-Y) of the supporting block (11, 11A, 11B).

4. Holder according to claim 1, characterised in that the retaining bracket (12)/the bearing bracket (13) includes two symmetrical clamping portions having a common central aperture.

5. Holder according to claim 1, characterised in that the retaining bracket (12)/the bearing bracket (13) includes a common bridge member having two apertures (12C, 13C) in its end regions.

6. Holder according to claims 1 to 5, characterised in that retaining bracket/bearing bracket is/are formed from resilient plastics material having a rectangular cross-section.

7. Holder according to claims 1 to 6, characterised in that the face of the retaining bracket/bearing bracket surrounding the apertures (12C, 13C) is bent upwardly or downwardly relative to the end regions, or respectively the bridge member.

8. Holder according to claims 5 and 7, characterised in that a retaining bracket (12) and a bearing bracket (13), each with a common bridge member, are mounted one above the other on two supporting blocks (11A, 11B), so that a space (Z) is formed for accommodating an object (70C) to be sterilised.

9. Holder according to claim 5 or claim 8, characterised in that the side of the retaining bracket or bearing bracket serving as a supporting face or retaining face is provided with pins (14) or raised dots (15).

10. Holder according to claim 1, characterised in that, for directional definition, the supporting block (11) and the retaining bracket (12) have corresponding guide means, such as, for example, a vertical elongate groove (16) and a guiding projection member (17) sliding therein.

## Revendications

1. Elément de support, en particulier pour la technique médicale, enfichable pour le support et la fixation d'un objet à stériliser, par exemple d'une trousse d'opération, dans un plateau de support lequel présente à cet effet un grand nombre d'ouvertures d'insertion pour une pluralité d'éléments de support pour des objets différents, **caractérisé en ce** qu'un élément de support est constitué par au moins un bloc de retenue (11, 11A, 11B) au moins partiellement profilé et par au moins un étrier de retenue (12, 12A, 12B) et/ou étrier de support (13), et que les étriers de retenue/étriers de support comportent au moins une ouverture (12C, 13C) à l'aide de laquelle ils peuvent être emboîtés sur au moins l'un des blocs de retenue (11, 11A, 11B) et que les arêtes de l'ouverture (12C, 13C) peuvent être verrouillées par encliquetage, sous déformation élastique de l'étrier de retenue/étrier de support, avec le profilage du (des) bloc(s) de retenue (11, 11A, 11B).

2. Elément de support selon la revendication 1, caractérisé en ce que le bloc de retenue (11, 11A 11B) est une pièce moulée de forme cylindrique sur la circonférence de laquelle sont conformées une pluralité de nervures d'arrêt (11D).

3. Elément de support selon la revendication 1, caractérisé en ce que la longueur intérieure (L) de l'ouverture (12C, 13C) de l'étrier de retenue/étrier de support est légèrement plus grande que la largeur intérieure (W) du bloc de retenue (11, 11A, 11B) dans le plan horizontal de telle façon que le blocage par encliquetage est obtenu lorsque le plan (X-X) de l'ouverture (12C, 13C) est incliné d'un angle ($\alpha$) différent de 90° par rapport à l'axe longitudinal (Y-Y) du bloc de retenue (11, 11A, 11B).

4. Elément de support selon la revendication 1, caractérisé en ce que l'étrier de retenue (12)-/l'étrier de support (13) est constitué par deux éléments d'étrier symétriques avec une ouverture centrale commune.

5. Elément de support selon la revendication 1, caractérisé en ce que l'étrier de retenue (12)-/l'étrier de support (13) est constitué par un élément de pont commun avec deux ouvertures (12C, 13C) dans ses sections terminales.

6. Elément de support selon l'une des revendications 1 à 5, caractérisé en ce que le(s) étrier(s) de retenue/étrier(s) de support est (sont) réalisé(s) dans une matière plastique élastique de section rectangulaire.

7. Elément de support selon l'une des revendications 1 à 6, caractérisé en ce que la surface de l'étrier de retenue/étrier de support entourant les ouvertures (12C, 13C) est coudée vers le haut et respectivement vers le bas par rapport aux sections terminales et respectivement à l'élément de pont.

8. Elément de support selon les revendications 5 et 7, caractérisé en ce qu'un étrier de retenue (12) et un étrier de support (13) sont emboîtés chacun avec un élément de pont commun en superposition sur deux blocs de retenue (11A, 11B) de façon à laisser un intervalle (Z) pour la réception d'un objet (70C) à stériliser.

9. Elément de support selon l'une des revendications 5 ou 8, caractérisé en ce que la face de l'étrier de retenue ou de l'étrier de support servant de surface d'appui ou de retenue est munie de tenons (14) ou de nopes (15).

10. Elément de support selon la revendication 1, caractérisé en ce que le bloc de retenue (11) et l'étrier de retenue (12) comprend pour la définition directionnelle des moyens de guidage correspondants, par exemple une encoche longitudinale (16) et un tenon de guidage (17) coulissant dans ladite encoche.

FIG.1

FIG.2

FIG.3

FIG.4

FIG.5

FIG.6

11 A

11 B

12

12C

14

12C

13C

Z
13C

70C

14

13

60

M

EP 0 283 928 B1